Europäisches Patentamt

European Patent Office

Office européen des brevets

(19)

(11) Numéro de publication: **0 232 299**
**B1**

(12) # FASCICULE DE BREVET EUROPEEN

(45) Date de publication du fascicule du brevet:
**21.02.90**

(51) Int. Cl.⁵: **A 61 L 15/00, D 04 H 1/64,**
**D 21 H 17/34**

(21) Numéro de dépôt: **86904223.4**

(22) Date de dépôt: **22.07.86**

(86) Numéro de dépôt international:
**PCT/FR 86/00257**

(87) Numéro de publication internationale:
**WO 87/00438 (29.01.87 Gazette 87/3)**

(54) **FIXATION DE POLYMERES RETENANT LES LIQUIDES DANS UNE STRUCTURE POREUSE.**

(30) Priorité: **23.07.85 FR 8511256**

(43) Date de publication de la demande:
**19.08.87 Bulletin 87/34**

(45) Mention de la délivrance du brevet:
**21.02.90 Bulletin 90/8**

(84) Etats contractants désignés:
**AT BE CH DE FR GB IT LI LU NL SE**

(56) Documents cités:
**EP-A- 0 036 463**
**EP-A- 0 077 510**
**FR-A- 2 245 671**
**GB-A- 2 083 487**
**US-A- 4 059 552**
**US-A- 4 235 237**

(73) Titulaire: **KAYSERSBERG SA, Route de Lapoutroie,**
**F-68240 Kaysersberg (FR)**

(72) Inventeur: **GOLDSTEIN, Guy, 3, rue Oberlin,**
**F-68000 Colmar (FR)**
Inventeur: **PIERRE, Michel, 2, passage de l'Hôtel de**
**Ville, F-68100 Mulhouse (FR)**

(74) Mandataire: **David, Daniel, KAYSERSBERG 54, avenue**
**Hoche, F-75008 Paris (FR)**

## Description

La présente invention concerne un procédé de fixation de polymères retenant les liquides, à l'intérieur d'un support présentant une structure poreuse ouverte, ainsi que le produit complexe obtenu selon ce procédé.

Les polymères retenant les liquides sont des macromolécules hydrophiles insolubles capables de former un gel en présence de liquide. On les appelle additifs à rétention améliorée (en abrégé ARA), hydrogels, hydrorétenteurs ou superabsorbants. Les produits les plus connus sont les alginates, par exemple ceux vendus sous la marque CECALGUM, les carboxyméthylcelluloses réticulés (marque AKUCELL de ENKA-AKZO), les amidons greffés (marque SAN-WET de SANYO CHEMICAL), les dérivés de synthèse du type acrylamide (marque AQUASORB de FLOERGER) ou du type acrylate (marques AQUAKEEP de SEITETSU, AQUALIC de NIPPON SHOKU-BAI ou ARASORB de ARAKAWA).

De nombreux procédés existent pour fixer la poudre sur ou dans un support. On peut coller la poudre au support par passage de vapeur, comme cela est décrit dans FR-A-2 066 324. Un dépôt de poudre de polymères peut être fixé aussi par pulvérisation de latex FR-A-2 402 474), ou bien emprisonné dans un enchevêtrement de fibres FR-A-2 446 357).

Le polymère peut être déposé sur les fibres par ensimage (brevet FR 2 283 255), sur un non-tissé EP-A-72 569) ou dans une mousse.

Le polymère peut être formé directement sur le support EP-A-123 500). La polymérisation est réalisée sous bombardement d'électrons des monomères déposés sur le support. Elle est obtenu en un temps très court limitant la formation de longues chaînes macromoléculaires. D'après le brevet cité ci-dessus, le polymère obtenu après bombardement électronique des monomères présente une capacité d'absorption d'eau salée d'environ 10 ml/g. De ce fait le complexe obtenu, pour être très absorbant, doit contenir une quantité importante de ce polymère, entre 3 et 12 fois son poids.

Dans le même domaine, JP-A-58.84804 décrit une méthode de polymérisation d'un mélange de monomères imprégnés dans un support poreux. Cependant la durée de la polymérisation est longue, elle limite la capacité de production. Par ailleurs, la réaction de polymérisation est conduite à température élevée, elle oblige à choisir des supports stables à ces températures.

Selon une autre méthode décrite dans GB-A-2 083 487, on immobilise des particules d'un polymère superabsorbant sur un support poreux en mettant les particules de polymère, initialement à l'état sec, en suspension dans un solvant organique, en appliquant ensuite la suspension sous forme de film liquide sur le support, et en éliminant enfin le solvant par séchage. Dans US-A-4 235 237 les particules de polymère sont gonflées et mises en suspension dans l'eau pour être appliquées ensuite sur le support.

L'invention a pour objet un nouveau procédé de dépôt de polymères superabsorbants sur un support, de mise en œuvre simple, et conduisant à un complexe présentant des propriétés améliorées.

Les polymères dont il est question dans l'invention sont des polymères obtenus par un procédé eau dans l'huile. Il s'agit d'un procédé d'émulsion inverse ou de suspension en phase réversible. Dans les procédés de ce type, la phase aqueuse contient les monomères et est dispersée dans une phase continue d'huile, c'est-à-dire d'un solvant non miscible à l'eau, au moyen d'un ou plusieurs éléments surfactifs généralement à caractère plus lipophile qu'hydrophile. On utilise de préférence des surfactifs à caractère non-ionique comme les esters de sorbitan.

Selon le procédé d'émulsion inverse, l'amorceur de polymérisation est généralement un peroxyde soluble dans le solvant, comme cela est décrit dans DE-C-1 089 173.

Selon les procédés de suspension en phase réversible, l'amorceur de polymérisation, qui est généralement du persulfate, est contenu dans la phase aqueuse. Cela est décrit par exemple dans EP-A-36 463 pour la fabrication du produit superabsorbant connu sous le marque AQUAKEEP ou dans DE-C-3 331 644 pour la fabrication du produit superabsorbant vendu sous la marque KAO SOAP.

Après la réaction, le polymère obtenu se retrouve dispersé dans le solvant en phase continue. Le solvant est généralement un hydrocarbure saturé, cyclique ou non, à raison de 5 à 50% en poids, plus précisément entre 10 et 25%. On utilise de préférence le cyclohexane ou l'heptane comme solvant. Après extraction et séchage, on obtient un polymère se présentant sous forme d'une poudre dont la qualité principale est d'être très absorbante.

Dans le procédé selon l'invention, on utilise l'émulsion ou la suspension inverse contenant le polymère, obtenu après la réaction de polymérisation. Le procédé est caractérisé en ce qu'il consiste à imprégner le support poreux avec ladite émulsion ou suspension inverse du polymère, résultant de la réaction de polymérisation, et à éliminer le solvant du support.

Par imprégnation, on entend l'action d'imbiber le support par l'émulsion ou la suspension, que ce soit par foulardage, par couchage, par filtration, par projection, par pulvérisation ou n'importe qu'elle autre technique équivalente.

Par élimination du solvant, on entend toute action qui permet de séparer le solvant du support poreux et du polymère. Ces actions telles que l'essorage, le séchage, l'évaporation sous vide partiel peuvent être utilisées seules, ou de façon complémentaire, voire simultanément.

Les polymères sont selon l'invention déposés dans le support, avant qu'ils n'aient subi aucune opération de séchage; c'est-à-dire préférentiellement immédiatement après, ou peu de temps après, leur fabrication, cette caractéristique est contraire au GB-A-2 083 487 où le polymère solide est remis en suspension dans un solvant organique et au US-A-4 235 237 où le polymère solide est gonflé avec de l'eau pour être appliqué au support.

L'imprégnation est réalisée sur un support poreux qui peut être un non-tissé, un papier, un voile de fibres, ou une mousse. Le choix du support sera guidé notamment par sa porosité, de préférence celle-ci sera supérieure à 0,5. En effet, les micelles de polymère en suspension ou émulsion dans le solvant migrent dans les pores si leur diamètre est inférieur à celui des pores. L'avantage d'utiliser la suspension ou l'émulsion au cours ou de préférence en fin de polymérisation réside dans le fait que les micelles sont microscopiques. Leur taille est généralement inférieure à 100 microns. On considère même que chaque micelle est constituée d'une seule macromolécule hydrophile à l'image d'une pelote de laine. Ces micelles, dont la taille est plus précisément de l'ordre de quelques dizaines de microns ou moins, sont capables de migrer dans des pores très fins comme ceux offerts par les structures compactées de fibres cellulosiques. On permet ainsi un éventail de choix de support, très large. De plus, suivant que l'on se trouve en présence d'un support poreux très ouvert ou très fermé, on peut augmenter ou diminuer la taille des micelles, en agitant ou non l'émulsion ou la suspension, en faisant varier la quantité du surfactif présent dans l'émulsion ou la suspension, ou bien en provoquant la floculation, par exemple par modification de la viscosité du mélange solvant-polymère. Dans ce dernier cas, l'addition d'eau peut suffire. Par ailleurs, en déstabilisant l'émulsion ou la suspension, l'addition d'eau favorise la précipitation du polymère. On réalise alors cette opération, de préférence avant l'imprégnation, en ajoutant l'eau au support.

Ainsi l'invention revient à séparer le polymère du solvant, par entraînement du polymère non séché à l'aide d'un support poreux. Ce dernier retient plus ou moins de polymères, si bien que la concentration en polymères dans le liquide imbibant le support est après imprégnation supérieure à celle de l'émulsion ou de la suspension avant l'imprégnation.

Si les micelles de polymères sont de taille trop importante par rapport aux pores du support, elles ne pénètrent pas dans le support. Le polymère se dépose essentiellement en surface sans être notablement fixé au support. Ce phénomène est courant lorsque l'on met un polymère solide en suspension dans un solvant, à l'exemple du brevet GB 2 083 487. En effet le solide obtenu par un procédé eau dans huile résulte de la coalescence de micelles. Les particules ainsi obtenues sont toujours d'une granulométrie plus élevés que les micelles. Quant aux solides obtenus par broyage, ils contiennent généralement peu de particules fines de moins de 100 microns. Un tamisage, opération coûteuse, s'impose pour pouvoir incorporer en milieu solvant une poudre dans un support dont les pores sont fins.

L'invention est illustrée par les exemples rapportés ci-après:

— le polymère utilisé est un polymère acrylate alcalin obtenu, selon la méthode décrit dans EP-A-36 463, en suspension inverse dans le cyclohexane à raison de 15% d'extraits secs.

— La méthode utilisée pour la détermination de la rétention, notée R30 par la suite, se décrit ainsi:

La rétention est mesurée en plaçant un échantillon de 0,5 g dans une serviette périodique de 8 g. La serviette contenant l'échantillon de poudre est immergée dans de l'eau salée contenant 10 g de chlorure de sodium par litre pendant 30 minutes. Elle est alors centrifugée à 1250 gravités pendant une minute. Par comparaison et différence entre une serviette avec échantillon et une sans échantillon, on évalue la rétention d'eau salée par gramme de polymère échantillon. La rétention, mesurée selon cette méthode, du polymère choisi pour les essais, est comprise entre 42 ml et 62 ml/g.

— Des essais ont été menés sur trois types de supports différents.

Le premier support est constitué d'une ouatine en fibres polyester thermoliées de 65 g/m², d'un volume massique de 35 cm³/g environ, sous une pression de 2,5 kPa. La porosité apparente, très élevée, est d'environ 0,97. La rétention R30 de ce support seul est pratiquement nulle: 0,02 g/g.

Un deuxième support est constitué d'un non-tissé, vendu sous la marque HOLNEST par BEGHIN-SAY, composé d'un voile de fibres de polyester (marque Hétérofil d'ICI) de 20 g/m² thermolié par calandrage selon un motif de losanges. Son volume massique est de 7 cm³/g. Le volume de vide est d'environ 6 cm³/g soit une porosité apparente de 0,85. La rétention R30 de ce support seul est d'environ 0,04 g/g.

Un troisième support est constitué d'un produit à base de fibres de cellules (85%) obtenu par voie sèche, renforcé par pulvérisation de latex (15% sec) sur ses deux faces. D'un grammage de 95 g/cm², avec un volume massique de 5 à 12 cm³/g selon les zones, ce produit d'essuyage fabriqué par BEGHIN-SAY présente une porosité totale comparable au non-tissé HOLNEST. Cependant les pores sont plus petits car le nombre de fibres par unités de volume est bien plus élevé. Ce support est encore moins perméable que les précédents aux grains de polymère à déposer. La rétention R30 est d'environ 1,5 g/g.

*Exemple 1*

La suspension de polymère mentionnée ci-dessus, est préparée dans un bac dans lequel on plonge le voile cardé en ouatine. Après imprégnation le voile est retiré et séché. Le tableau 1 résume les résultats obtenus suivant que le voile est séché horizontalement sur une plaque en Teflon (ligne a et b) ou verticalement (ligne c), ou bien que le support a été préalablement imprégné de solvant (ligne d), ou d'eau (ligne e, à raison de 21%, ligne f, à raison de 31%).

Les colonnes du tableau donnent le poids du support seul, sec ou mouillé dans le cas de la préimprégnation, le poids du complexe obtenu après imprégnation et séchage, le poids de l'ARA sec fixé sur le support après imprégnation en grammes et par rapport au support seul (X), enfin la rétention R30 telle quelle a été mesurée pour le complexe d'une part, ramenée à l'ARA contenu dans le complexe d'autre part.

TABLEAU 1

| CONDITIONS OPERATOIRES Exemples 1 | SUPPORT | | COMPLEXE (SEC) | ARA | | RETENTION R30 | |
|---|---|---|---|---|---|---|---|
| | SEC | MOUILLE | | MASSE m(g) | X | COMPLEXE (g/g) | ARA (g/g) |
| Voile cardé PET sec Séchage horizontal | | | | | | | |
| a | 1,11 g | | 8,87 g | 7,76 g | X 7 | 15 | 17 |
| b | 1,16 g | | 8,78 g | 7,62 g | X 6,5 | 15 | 17 |
| Séchage vertical | | | | | | | |
| c | 2,05 g | | 11,25 g | 10,20 g | X 5 | 13 | 14,5 |
| Voile + cyclohexane | 0,93 g | 1,03 g (11%) | 5,30 g | . 4,37 g | X 4,5 | 13,5 | 16 |
| Voile + eau | | | | | | | |
| e | 0,78 g | 0,99 g (21%) | 4,30 g | 3,52 g | X 4,5 | 13 | 16 |
| f | 0,89 g | 1,17 g (31%) | 6,70 g | 5,81 g | X 6,5 | 14,5 | 17 |

On constate ainsi que selon cette méthode le complexe obtenu retient en eau plus de 10 fois son propre poids, de 13 à 15 fois.

*Exemple 2*

La même suspension sert à l'imprégnation du non-tissé (marque Holnest) décrit ci-dessus.

Ce support est moins poreux que la ouatine; on constate que le dépôt d'additif à rétention améliorée (ARA) est moins important: 3 à 4 g/g. En humidifiant le non-tissé avant l'imprégnation, on fixe une quantité d'ARA légèrement plus importante (ligne b) qu'en l'absence d'eau (ligne a du tableau 2).

TABLEAU 2

| CONDITIONS OPERATOIRES Exemples 2 | SUPPORT | | COMPLEXE (SEC) | ARA | | RETENTION R30 | |
|---|---|---|---|---|---|---|---|
| | SEC | MOUILLE | | MASSE m(g) | X | COMPLEXE (g/g) | ARA (g/g) |
| NT Holnest sec | | | | | | | |
| a | 0,27 | | 1,10 g | 0,83 g | X 3,1 | 14,5 | 19 |
| NT Holnest + eau | | | | | | | |
| b | 0,59 | 1,43 g (42%) | 2,84 g | 2,25 g | X 3,8 | 14,5 | 18 |

*Example 3*

Une nouvelle suspension de polymère est préparée, toujours selon le même procédé de suspension inverse, les paramètres sont choisis afin d'obtenir un polymère à caractéristiques de rétention supérieures à celles des exemples 1 et 2.

Les essais sont menés sur des échantillons de ouatine de 5 cm de laize. Les échantillons sont déroulés sur un cylindre perforé tournant dans une cuve de 100 ml.

On constate que le taux de polymère déposé est moindre car les micelles de la suspension sont plus petites. Avec un taux d'ARA déposé de 1 à 2,5 g/g et une rétention du complexe de 20 ml/g, la rétention R30 de l'ARA est estimée entre 25 et 40 g/g. Voir le tableau 3.

TABLEAU 3

| ESSAIS(N) 5 × 40 cm | SUPPORT (g) | SUPPORT IMPREGNE g | X | COMPLEXE (g) | ARA / SUPPORT m(g) | X | RETENTION R30 Complexe | ARA |
|---|---|---|---|---|---|---|---|---|
| a | 1,35 | 12,8 | 8,5 | 2,90 | 1,55 | 1,1 | 21,3 | 40 |
| b | 1,25 | 12,9 | 9,3 | 3,20 | 1,95 | 1,6 | 20,3 | 33 |
| c | 1,30 | 16,6 | 11,7 | 4,57 | 3,27 | 2,5 | 20,7 | 29 |
| d | 1,20 | 13,6 | 10,3 | 3,06 | 1,86 | 1,5 | 15 | 25 |
| e | 1,30 | 13,7 | 9,5 | 3,64 | 2,34 | 1,8 | 17,5 | 27 |
| f | 1,40 | 12,9 | 8,2 | 3,09 | 1,69 | 1,2 | 16 | 29 |
| g | 1,30 | 12,9 | 8,9 | 3,57 | 2,27 | 1,7 | 18 | 28 |

*Exemple 4*

La même suspension qu'en l'exemple 3 est utilisée.

La ouatine est pré-imprégnée d'eau. La quantité d'ARA déposé augmente avec la quantité d'eau imbibant le voile polyester. Les performances de l'ARA diminuent de 40 g/g (3a) à 17 g/g (4f) en fonction du taux d'ARA déposé. L'eau d'imprégnation (4a, 4b, 4c, 4d, 4e) sert à augmenter la précipitation du polymère sur le support par destabilisation du polymère en suspension inverse.

On constate que la quantité d'eau optimale pour mouiller le support en polyester est présentement de 50 à 100%. Au-delà on n'obtient plus de différence significative. En entraînant plus de polymère, l'émulsion ou la suspension s'épuise.

L'homme de l'art recherchera à obtenir le meilleur produit final à partir d'émulsion et de suspension très fines destabilisées dans des supports très poreux mais à surface spécifique élevée pour diviser au maximum le polymère déposé.

TABLEAU 4

| ESSAIS | SEC (g) | SUPPORT MOUILLE (g) (%H₂O) | SUPPORT IMPREGNE (g) | COMPLEXE SEC (g) | ARA m (g) | X | RETENTION R30 (g/g) COMPLEXE | ARA |
|---|---|---|---|---|---|---|---|---|
| a | 1,34 | 1,47 (10) | 16,0 | 4,47 | 3,13 | 2,3 | 22 | 31,5 |
| b | 1,50 | 1,87 (24) | 20,9 | 5,65 | 4,15 | 2,8 | 22 | 30 |
| c | 1,59 | 2,29 (44) | 37,3 | 8,55 | 6,96 | 4,4 | 21,5 | 26,5 |
| d | 1,64 | 6,2 (338) | 39,5 | 10,40 | 8,76 | 5,3 | 20,5 | 24 |
| e | 1,35 | 5,7 (320) | 31,0 | 7,73 | 6,38 | 4,7 | 15,7 | 19,0 |
| f | 1,36 | 7,2 (430) | 34,5 | 7,40 | 6,04 | 4,4 | 15 | 18,4 |
| g | 1,22 | 6,4 (420) | 33,8 | 7,70 | 6,48 | 5,3 | 17 | 20,2 |
| h | 1,24 | 7,2 (480) | 25,6 | 5,33 | 4,09 | 3,3 | 16,3 | 21,2 |

*Exemple 5*

Une nouvelle suspension inverse d'acrylate de sodium dans du cyclohexane, stabilisée par la même quantité de surfactif que précédemment est préparée.

Une partie sert à l'imprégnation de ouatine, une autre partie est traitée pour obtenir 90 g de polymère solide dont les particules sont très fines. La granulométrie moyenne est de 100 microns. La rétention R30 est de 45 à 52 g/g.

Trois types d'essays dont les résultats sont repris dans le tableau 5 ont été réalisés.

TABLEAU 5

| ESSAIS | SUPPORT SEC (g) | MOUILLE (g) | SUPPORT IMPREGNE (g) | COMPLEXE SEC (g) * ** | ARA/SUPPORT (g) * ** | RETENTION COMPLEXE ** g/g |
|---|---|---|---|---|---|---|
| 5a A | 0,35 | | 3,27 | 0,89 - 0,88 | 0,54 - 0,53 | 25 |
| B | 0,36 | | 3,25 | 0,89 - 0,72 | 0,53 - 0,36 | 23 |
| C | 0,36 | | 3,56 | 0,89 - 0,97 | 0,53 - 0,61 | 26 |
| 5b D | 0,37 | | 2,75 | 0,89 - 0,45 | 0,52 - 0,08 | 4 |
| E | 0,33 | | 3,03 | 1,20 - 0,59 | 0,87 - 0,26 | 18 |
| F | 0,38 | | 2,48 | 0,60 - 0,47 | 0,22 - 0,09 | 5 |
| G | 0,30 | | 1,66 | 0,50 - 0,39 | 0,20 - 0,09 | 6 |
| H | 0,36 | | 2,64 | 0,69 - 0,49 | 0,32 - 0,13 | 7 |
| I | 0,30 | | 1,66 | 0,41 - 0,40 | 0,11 - 0,10 | 7 |
| 5c J | 0,34 - 0,75 | | 3,06 | 0,67 - 0,59 | 0,33 - 0,25 | 21 |
| K | 0,38 - 0,77 | | 3,61 | 0,81 - 0,73 | 0,43 - 0,35 | 16 |
| L | 0,39 - 0,90 | | 4,88 | 1,11 - 0,68 | 0,72 - 0,29 | 20 |

\* Après séchage
\** Après séchage et manipulation des échantillons; c'est-à-dire avant analyse.

— *essai 5a*

La ouatine est imprégnée de la suspension inverse.

Les complexes A, B et C contiennent 1,5 g de polymère par gramme de ouatine, répartis à l'intérieur de la ouatine. Ils retiennent 23 à 26 grammes d'urine synthétique par gramme de complexe. Le polymère déposé retient 40 à 45 g/g environ. Il est déposé et fixé dans la ouatine. La manipulation des échantillons n'entraîne pas de perte de polymère non fixé.

— *essai 5b*

40 g de polymère solide obtenu sont mis en suspension dans 225 g de cyclohexane sous reflux à la température de 50°C contenant le même surfactif dans la même proportion que celle définie lors de sa synthèse. La suspension est particulièrement instable. L'imprégnation des échantillons de ouatine est également réalisée avec la suspension sous agitation. Après séchage, on constate que le polymère n'est pas bien fixé à ce support très poreux. Le polymère s'est déposé dans les alvéoles de la ouatine. Il n'est pas fixé comme le montrent les pertes de poids entre les pesées avant analyse et celles après séchage (troisième et quatrième colonnes du tableau 5). Ce procédé différent de celui revendiqué est moins efficace.

Les rétentions des complexes D à I sont faibles car la quantité de polymères présent au moment de l'analyse est minime.

— *essai 5c*

A la différence des essais 5b, la ouatine est mouillée par de l'eau (100% d'eau environ) avant d'être plongée dans la suspension. Le polymère solide est mieux fixé, les pertes sont moindres. La qualité du complexe (rétentions plus faibles) est inférieure, son coût dû aux séchages du polymère puis du complexe est cependant supérieur à celui obtenu dans l'exemple 5a selon l'invention.

*Exemples 6*

Le support poreux utilisé est le produit mentionné plus haut, obtenu par voie sèche. Les pores de ce support sont plus étroits que ceux de la ouatine.

Trois types d'essais ont été menés, les résultats sont repris dans le tableau 6.

— *essai 6a*

Ce support plongé dans la suspension inverse comme pour les essais 5a s'en imbibe. Le complexe avant analyse (**), est plus lourd car il contient 8% d'eau par rapport au complexe pesé après séchage (*). Comme le montrent les échantillons A, B, C, D, il n'y a pas de perte de polymère. Le polymère est bien fixé au support.

La rétention du polymère est d'environ 40 à 50 g/g comme à l'exemple 5a.

Ces exemples 5a et 6a illustrent bien l'invention.

TABLEAU 6

| ESSAIS | | SUPPORT SEC (g) | SUPPORT IMPREGNE | COMPLEXE SEC (g) * ** | ARA/SUPPORT ** | RETENTION COMPLEXE ** |
|---|---|---|---|---|---|---|
| 6a | A | 0,48 | 4,70 | 0,84 - 0,99 | 0,51 | 20 g/g |
| | B | 0,44 | 4,51 | 0,86 - 0,93 | 0,49 | 20 |
| | C | 0,42 | 4,51 | 0,84 - 0,90 | 0,48 | 25 |
| | D | 0,44 | 4,62 | 0,90 - 0,97 | 0,53 | 22 |
| 6b | E | 0,44 | 4,14 | 0,83 - 0,68 | 0,24 | 13 |
| | F | 0,45 | 4,05 | 0,89 - 0,87 | 0,42 | 18 |
| | G | 0,45 | 3,99 | 0,84 - 0,76 | 0,31 | 14 |
| | H | 0,43 | 3,90 | 0,87 - 0,81 | 0,38 | 19 |
| | I | 0,43 | 4,19 | 0,80 - 0,65 | 0,22 | 14 |
| | J | 0,42 | 3,50 | 0,63 - 0,63 | 0,21 | 14 |
| 6c | K | 0,42 | 4,58 | 0,77 - 0,77 | 0,35 | 22 |
| | L | 0,45 | 5,16 | 0,99 - 0,95 | 0,50 | 20 |
| | M | 0,46 | 5,51 | 1,00 - 0,98 | 0,52 | 20 |
| | N | 0,47 | 4,26 | 0,62 - 0,62 | 0,15 | 8 |
| | O | 0,44 | 5,95 | 1,22 - 1,30 | 0,66 | 19 |
| | P | 0,44 | 6,68 | 1,49 - 1,67 | 1,25 | 21 |

\* Après séchage
\*\* Avant analyse

— *essais 6b*

Le support est imprégné de la suspension de l'exemple 5b. Le dépôt de polymère est équivalent. Cependant on constate que la poudre s'est déposée en surface du support sous forme de croûte qui se désagrège en manipulant le complexe.

Dans ce support moins poreux que la ouatine, le polymère remis en suspension ne pénètre presque plus comme on a pu le voir sur les échantillons E, F, G, H, I, J.

— *essais 6c*

Le support est imprégné de la suspension de l'exemple 5b dans laquelle 40 g de cyclohexane ont été remplacés par 40 g d'eau. La fixation de polymère est correcte car le polymère légèrement gélifié colle au support. Mais le polymère gonflé ne pénètre pratiquement pas dans ce support moins poreux que la ouatine, l'examen des échantillons K, L, M, N, O et P montre qu'il reste nettement en surface.

La mise en suspension d'un polymère solide dans un solvant porteur puis l'imprégnation d'un support poreux et enfin l'élimination du solvant sont des opérations connues. (5b, 5c, 6b, 6c). Elles ne permettent cependant pas de réaliser des complexes d'aussi bonne qualité que le procédé revendiqué où le polymère qui n'a jamais été séché est séparé du solvant alors qu'il est contenu dans le support poreux. Le produit obtenu se caractérise en ce qu'il comporte au moins 0,45 gramme de polymère insoluble hydrophile par gramme de support. Sa rétention R30 est au moins égale à 10 ml par gramme de produit.

Les marques commerciales deposées sont reconnues comme telle.

**Revendications**

1. Procédé de dépôt et fixation de polymère insoluble hydrophile dans ou sur un support poreux, ledit polymère étant obtenu selon un procédé «eau dans huile» en émulsion ou suspension inverse, caractérisé en ce qu'il consiste à imprégner le support poreux avec ladite émulsion ou suspension inverse de polymère, résultant de la réaction de polymérisation et à éliminer le solvant du support.

2. Procédé selon la revendication 1, caractérisé en ce que l'invention ou la suspension inverse est déstabilisée pendant l'opération d'imprégnation afin de faciliter le dépôt du polymère sur le support poreux.

3. Procédé selon la revendication 2, caractérisé en ce que l'émulsion ou la suspension inverse est déstabilisée par addition d'eau durant l'imprégnation.

4. Procédé selon la revendication 3, caractérisé en ce que l'addition d'eau est réalisée par imprégnation du dupport poreux, avant son imprégnation avec l'émulsion ou la suspension inverse.

5. Procédé selon la revendication 1, caractérisé en ce que l'émulsion ou la suspension inverse comprend entre 10% et 30% de polymère hydrophile.

6. Procédé selon la revendication 1, le support poreux étant constitué d'un non-tissé, d'un papier, d'un matelas de fibres cellulosiques et/ou synthétiques lié ou non lié ou d'une mousse, caractérisé en ce que ledit support présente une porosité supérieure à 0,5.

7. Produit obtenu selon l'une des revendications précédentes, caractérisé en ce qu'il comporte au moins 0,45 grammes de polymère insoluble hydrophyle par gramme de support.

8. Produit selon la revendication 7, caractérisé en ce que sa rétention R30 est au moins égale à 10 ml par gramme de produit.

## Patentansprüche

1. Verfahren zur Ablagerung und Fixierung von unlöslichem, hydrophilen Polymer in oder auf einem porösen Träger, wobei das Polymer gemäß eines «Wasser-in-Öl-Verfahrens» in einer Emulsion oder in inverser Suspension erhalten wird, dadurch gekennzeichnet, daß dabei der poröse Träger mit der Emulsion oder inversen Suspension des Polymers imprägniert wird, das aus der Polymerisationsreaktion stammt und daß das Lösungsmittel vom Träger entfernt wird.

2. Verfahren nach Anspruch 1, dadurch gekennzeichnet, daß die Emulsion oder die inverse Suspension während des Imprägnierungsverfahrens destabilisiert werden, um die Ablagerung des Polymers auf dem porösen Träger zu erleichtern.

3. Verfahren nach Anspruch 2, dadurch gekennzeichnet, daß die Emulsion oder die inverse Suspension durch Wasserzugabe während der Imprägnierung destabilisiert wird.

4. Verfahren nach Anspruch 3, dadurch gekennzeichnet, daß die Wasserzugabe durch Imprägnierung des porösen Trägers bewirkt wird, vor seiner Imprägnierung mit der Emulsion oder der inversen Suspension.

5. Verfahren nach Anspruch 1, dadurch gekennzeichnet, daß die Emulsion oder die inverse Suspension zwischen 10% und 30% des hydrophilen Polymers enthalten.

6. Verfahren nach Anspruch 1, bei dem der poröse Träger ein Vlies, ein Papier, eine aus verbundenen oder nicht verbundenen Cellulosefasern und/oder synthetischen Fasern bestehende Matte oder ein Schaum ist, dadurch gekennzeichnet, daß der Träger eine Porosität oberhalb von 0,5 aufweist.

7. Produkt, hergestellt nach einem der vorhergehenden Ansprüche, dadurch gekennzeichnet, daß es mindestens 0,45 g unlösliches, hydrophiles Polymer pro Gramm Trägermaterial enthält.

8. Produkt nach Anspruch 7, dadurch gekennzeichnet, daß sein Rückhaltevermögen R30 mindestens gleich 10 ml pro Gramm Produkt ist.

## Claims

1. Process for the deposition and fixation of insoluble hydrophilic polymer in or on a porous support, the said polymer being obtained according to a «water-in-oil» process in emulsion or reverse suspension, characterized in that it consists in impregnating the porous support with the said emulsion or reverse suspension of polymer resulting from the polymerization reaction, and in removing the solvent from the support.

2. Process according to Claim 1, characterized in that the emulsion or the reverse suspension is destabilized during the impregnation operation in order to facilitate the deposition of the polymer on the porous support.

3. Process according to Claim 2, characterized in that the emulsion or the reverse suspension is destabilized by addition of water during impregnation.

4. Process according to Claim 3, charecterized in that the addition of water is carried out by impregnation of the porous support, before impregnation with the emulsion or the reverse suspension.

5. Process according to Claim 1, characterized in that the emulsion or the reverse suspension contains between 10% and 30% of hydrophilic polymer.

6. Process according to Claim 1, the porous support comprising a nonwoven, a paper, a padding of cellulose and/or synthetic fibres which may be bonded or unbonded, or a foam, characterized in that the said support has a porosity greater than 0.5.

7. Product obtained according to one of the preceding claims, characterized in that it contains at least 0.45 grams of insoluble hydrophilic polymer per gram of support.

8. Product according to Claim 7, charactereized in that its retention R30 is at least equal to 10 ml per gram of product.